# EUROPEAN PATENT APPLICATION

(11) **EP 0 658 550 A2**
(43) Date of publication of application: **21.06.1995**
(21) Application number: 94119404.5
(22) Date of filing: 08.12.1994
(51) Int. Cl.: C07D 257/04, C07D 405/06

(54) **Amino acid salts of and methods for preparing antihypercholesterolemic tetrazole compounds**

(30) Priority: 15.12.1993 US 168007
(71) Applicant: BRISTOL-MYERS SQUIBB COMPANY, Princeton, NJ 08543-4000 (US)
(72) Inventor: Thottathil, John K., Robbinsville, NJ 08691 (US); Pendri, Yadagiri, Matawan, NJ 07747 (US); Li, Wen-Sen, Marlboro, NJ 07746 (US); Venit, John J., East Brunswick, NJ 08816 (US); Kiang, San, Madison, NJ 07940 (US); Waltermire, Robert, Wilmington, DE 19803 (US)
(74) Representative: Josif, Albert, Dr.-Ing.

(57) **Abstract**

Compounds of the formula 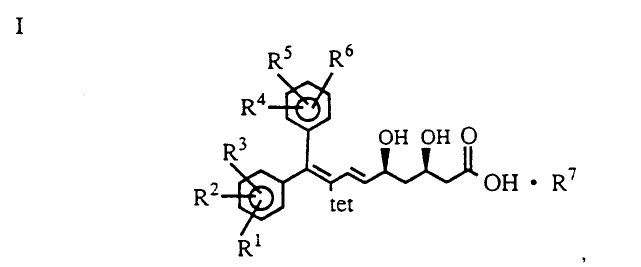
wherein R¹ and R⁴ each are independently hydrogen, halogen, C_{1 -4} alkyl, C_{1 -4} alkoxy or trifluoromethyl; R², R³, R⁵ and R⁶ each are independently hydrogen, halogen, C_{1 -4} alkyl or C_{1 -4} alkoxy; R⁷ is an amino acid such as arginine or lysine; and tet is 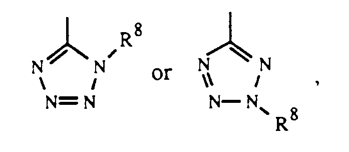
where R⁸ is hydrogen, C₁₋₄ alkyl, C₁₋₄ alkoxyalkyl or (2-methoxy-ethoxy)methyl; methods of preparing compouds of formula 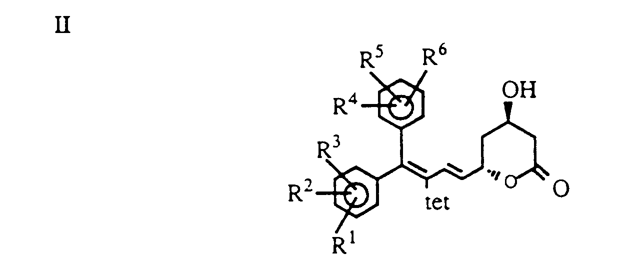
and intermediates. The compounds of formula I and II are inhibitors of the enzyme 3-hydroxy-3-methyl-glutaryl coenzyme A (HMG-CoA) reductase and are useful in the treatment of hypercholesterolemia, hyper- lipoproteinemia and atherosclerosis.

## Description

The present invention is directed to the amino acid sets such as the L-arginine or lysine salts of the compounds of formula 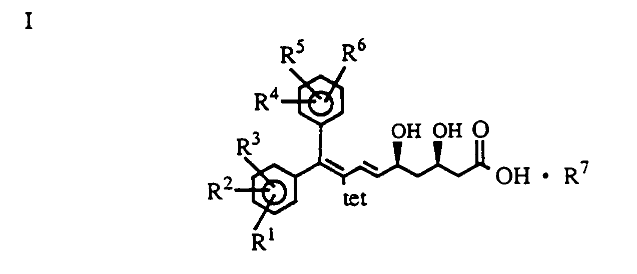
wherein R¹ and R⁴ are independently hydrogen, hydrogen, C₁ -₄ alkyl, C₁ -₄ alkoxy or trifluoromethyl; R², R³, R⁵ and R⁶ are independently hydrogen, halogen, C₁ -₄ alkyl or C₁ -₄ alkoxy; R⁷ is an amino acid; and tet is 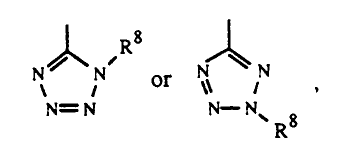
where R⁸ is hydrogen, C₁ -₄ alkyl, C₁ -₄ alkoxyalkyl or (2-methoxy-ethoxy)methyl.

The invention also includes a novel process for preparing the lactones of formula

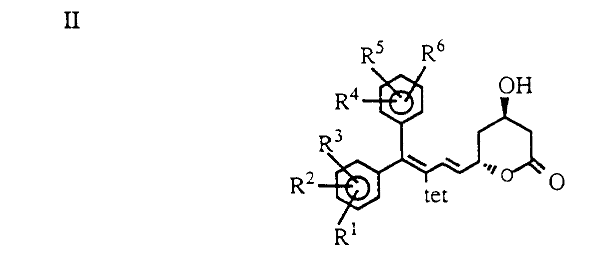

The invention also includes the crystalline hydrated species having the formula 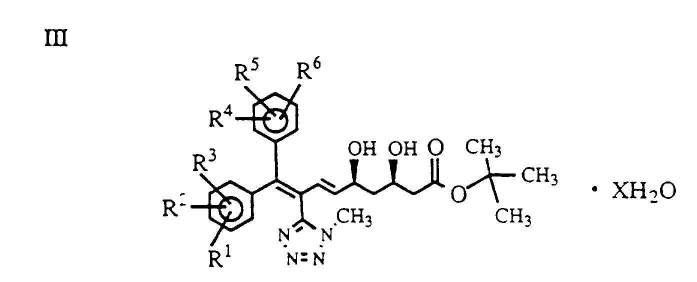
wherein X is preferably between about 0.45 to 0.65.

Further, the invention includes a process for the continuous preparation of compounds of the formula 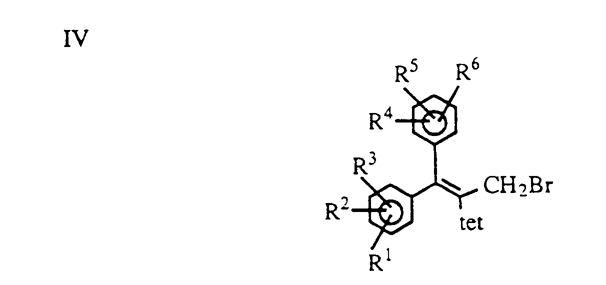
including the steps of (a) heating a solution having a compound of formula 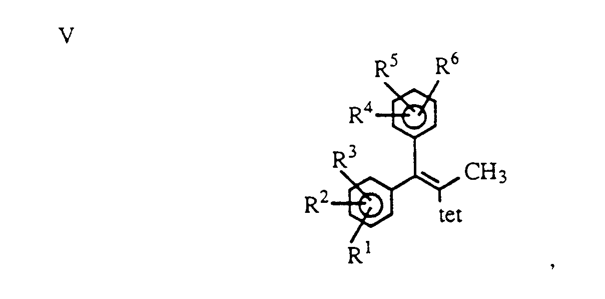
a brominating agent and a free radical initiator in a halogenated hydrocarbon solvent to a temperature of between about 30 to 35 _{°} C; and (b) subsequently exposing the heated solution to UV or visible light.

The compounds of formula I and II are potent inhibitors of the enzyme 3-hydroxy-3-methylglutaryl coenzyme A (HMG-CoA) reductase and are useful in the treatment of hypercholesterolemia, hyper- lipoproteinemia and atherosclerosis. The compounds of formula III and IV are intermediates useful in the preparation of compounds of formula I and II.

The present invention provides the amino acid salts of the formula I, compounds of formula III and methods of preparing the compounds of formula I, II and IV. Listed below are definitions of various terms used to describe the compounds of the instant invention. These definitions apply to the terms as they are used throughout the specification (unless they are otherwise limited in specific instances) either individually or as part of a larger group.

The terms "C₁₋₄ alkyl", "C₁₋₆ alkyl" and "C₁₋₄ alkoxy" refer to unbranched or branched chain alkyl or alkoxy groups such as methyl, ethyl, propyl, isopropyl, butyl, isobutyl, t-butyl, amyl, hexyl, etc. Preferably, these groups contain from 1 to 4 carbon atoms and, most preferably, they contain 1 or 2 carbon atoms.

The term "halogen" or "halide" as used herein refers to chlorine, fluorine, bromine and iodine.

In the compounds of formula I and II, it is intended that all of the double bonds are in the trans configuration. i.e., (E), as indicated in the structural formulae used herein and in the claims.

As the compounds of the present invention may possess one or two asymmetric carbon atoms, the invention includes all of the possible enantiomeric and diastereomeric forms of the compounds of formula I and II as described herein and in the claims. The compounds of formula I and II which contain two centers of asymmetry may produce four possible stereoisomers designated as the RR, RS, SR and SS enantiomers; all four stereoisomers are considered within the scope of this invention. Specifically, the compounds of formula I having two asymmetric carbon atoms bearing the hydroxy groups in the 3 and 5 position may produce four possible stereoisomers which are designated as the (3R, 5S), (3S, 5R), (3R, 5R) and (3S, 5S) stereoisomers. As used herein and in the claims, the term "(±)-erythro" is intended to include a mixture of (3R, 5S) and (3S, 5R) enantiomers, and the term "(±)-threo" is intended to include a mixture of (3R, 5R) and (3S, 5S) enantiomers. The use of a single designation such as (3R, 5S) is intended to include mostly one stereoisomer. The lactone compounds of formula II also have two asymmetric carbon atoms at the 4 and 6 position, and the resulting four stereoisomers may be designated as the (4R, 6S), (4S, 6R), (4R, 6R) and (4S, 6S) stereoisomers. As used herein and in the claims, the term "trans" lactone is intended to include a mixture of (4R, 6S) and (4S, 6R) enantiomers while the term "cis" lactone is intended to include a mixture of (4R, 6R) and (4S, 6S) enantiomers. The use of a single designation such as (4R, 6S) is intended to include mostly one stereoisomer. Mixtures of isomers can be separated into individual isomers according to methods which are known in the art, e.g. fractional crystallization, adsorption chromatography or other suitable separation processes.

Since the compounds of formula I and II appear to contain varying amounts of solvent as ascertained mainly by elemental analysis, the present invention is intended to include solvates of the compounds of formula I and II. In some cases, it appears that the products may be true solvates, while in other cases, the products may merely retain adventitious solvent or be a mixture of solvate plus some adventitious solvent. Preferably, the solvate is water and, most preferably, one to three moles of water. The examples below give the amount of solvent where appropriate in the analysis and melting points are those of the solvated product unless otherwise indicated.

Compound III is a descreet crystalline hydrate as ascertained mainly by Karl Fischer titration and elemental analysis, the present invention is intended to include this crystalline hydrated form. Typically, 0.45 to 0.65 moles of water are found in the crystalline form of the compound. The examples below give the amount of water where appropriate in the analysis and melting points are those of the solvated product unless otherwise indicated.

Preferably, in the compounds of formula I and II, the substituents R¹, R², R³, R⁵ and R⁶ are independently hydrogen, halogen, C₁ -₄ alkyl or C₁ -₄ alkoxy. More preferably, R¹ and R⁴ are hydrogen and R², R³, R⁵ and R⁶ are independently hydrogen, fluoro, chloro, methyl or methoxy, and most preferably, R₁ and R₄ are hydrogen and R², R³, R⁵ and R⁶ are independently hydrogen, fluoro, methyl or methoxy. Preferably, tet is 1 H-tetrazol-5-yl or 1-substituted-1H-tetrazol-5-yl. More preferably, tet is 1-methyl-1H-tetrazol-5-yl, 1-ethyl-1 H-tetrazol-5-yl, 1-methylethyl-1H-tetrazol-5-yl or 1-(2-methoxyethoxy)-methyl-1H-tetrazol-5-yl and most preferably, tet is 1-methyl-1 H-tetrazol-5-yl.

In the compounds of formula I wherein two asymmetric carbon atoms bearing the hydroxy group, the erythro isomer is preferred and the (3R, 5S) isomer is most preferred. In the compounds of formula II, where two asymmetric carbon atoms are present in the lactone ring, the trans isomer is preferred and (4R, 6S) isomer is most preferred.

The amino acid salts of the instant invention (compounds of formula I) are prepared from the free acids of formula 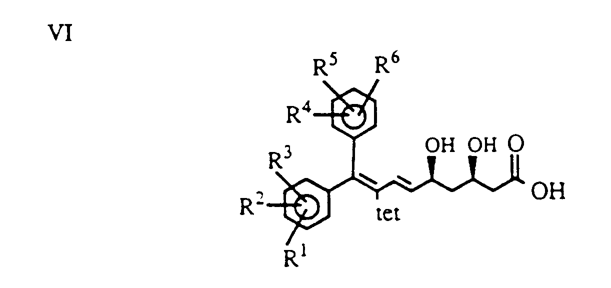
where R¹ to R⁶ and tet are as defined for formula I, by adding the free acid in an organic solvent (such as an ethyl acetate, methyl t-butyl ether or diethyl ether) to a slurry of L-arginine or lysine in an alcoholic solvent such as ethanol or methanol and about 8 to 16% water, preferably 8 to 13% water for the L-arginine and about 1.5 to 6.5% water for the lysine salt.

The free acids of formula VI may be prepared as disclosed in U.S. Patent No. 4,897,490, issued January 30, 1990, the disclosure of which is incorporated by reference herein.

The invention also comprises a novel method for preparing compounds of formula II. Compounds of formula II may be prepared by reacting a compound of formula 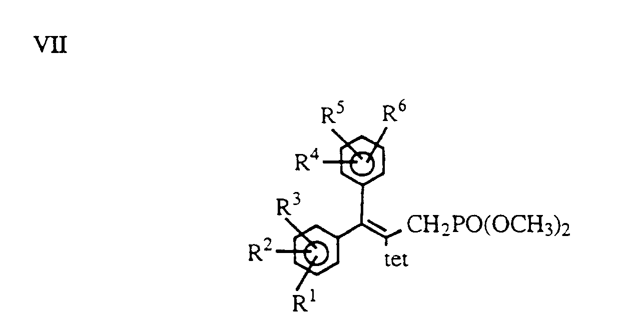
with a compound of formula 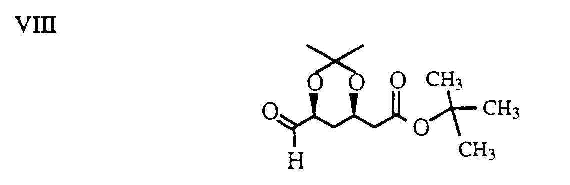
in the presence of a condensation agent such as a base, for example, KO-tAm or KO-t-Bu or similar suitable bases in an organic solvent such as tetrahydrofuran, to form compounds of formula

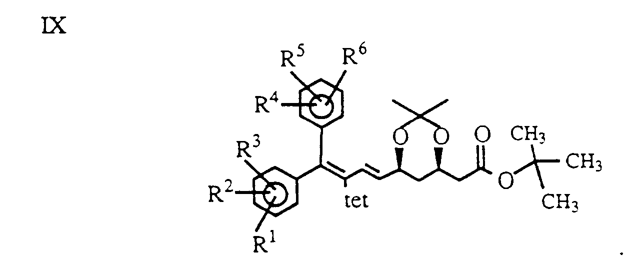

Compounds of formula IX are then converted to a compound of formula 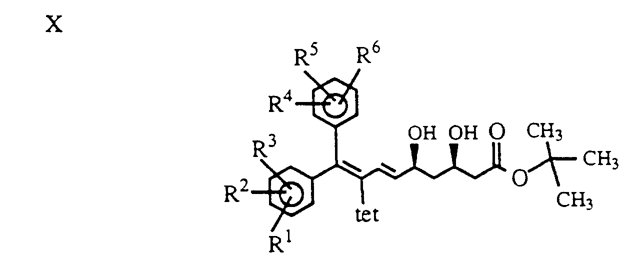
by reaction with an acid such as hydrochloric acid in a solvent such as methanol. Compounds of formula X are then reacted with a base such as sodium hydroxide followed by acidification with an acid such as hydrochloric acid to form the free acid of formula VI. The free acid is then reacted with a cinchonidine alkaloid in a suitable solvent such as ethyl acetate to form the compounds of formula

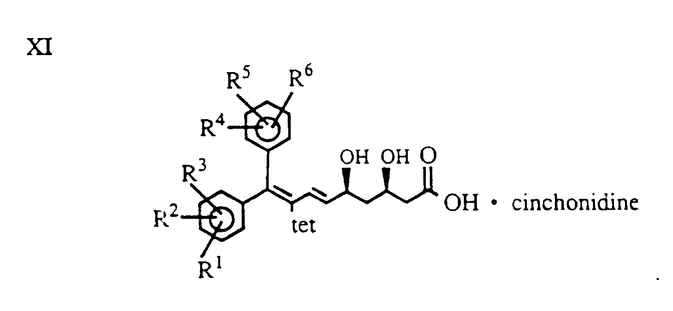

The cinchonidine salts of formula XI are then reacted with a coupling reagent or an activating agent such as pivaloyl chloride or DCC to form the compounds of formula II.

Compounds of formula III are crystallized as hydrated species by exchanging the solvent, such as methanol, of compounds of formula X with a higher boiling solvent such as methyl isobutyl ketone followed by concentration and dilution with heptane and water. Crystallization occurs upon cooling the mixture. If further purification is desired, compounds of formula III are recrystallized by dissolution in isopropanol/water, dilution with heptane followed by crystallization on cooling.

The arginine or lysine salt of formula I may also be prepared by reacting compounds of formula II, III or X with a base such as sodium hydroxide followed by acidification using for example, hydrochloric acid, to form the free acid of formula VI and then reacting with the appropriate amino acid such as arginine or lysine.

Alternatively, the arginine or lysine salt of formula I may be prepared by reacting the cinchonidine salt of formula XI with an acid such as hydrochloric acid to form the free acid of formula VI and then reacting with the appropriate amino acid such as arginine or lysine.

The starting materials of formulae VII and VIII are disclosed in U.S. patent 4,897,490.

Further, the invention includes a process for the continuous preparation of compounds of the formula 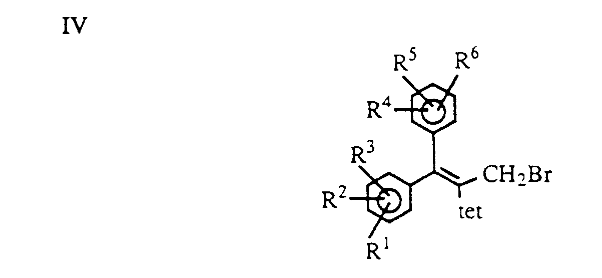
including the steps of (a) heating a solution having a compound of formula 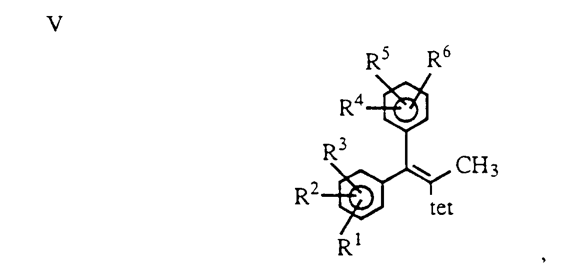
a brominating agent and a free radical initiator in a halogenated hydrocarbon solvent to a temperature of between about 30 to 35 °C; and (b) subsequently exposing the heated solution to UV or visible light. Exemplary brominating agents include N-bromosuccinimate (NBS) and 1,3-bromo-5,5-dimethylhydantoin. A suitable radical initiator is di(4-tert-butylcyclohexyl)peroxydicarbonate, commercially available as Perkadox@. Examples of halogenated hydrocarbon solvents include methylene chloiride, chlorobenzene, chloroform, carbon tetrachloride and toluene. The continuous preparation as described above allows for a safer reaction with high yields.

Compounds of formula IV are intermediates useful in the preparation of compounds of formula I and II. Additional methods for preparing compounds of formula IV and methods of converting compounds of formula IV to compounds of formula I and II are disclosed in U.S. patent 4,897,490.

For therapeutic use, the pharmacologically active compounds of formula I and II will normally be administered as a pharmaceutical composition comprising as the essential active ingredient at least one such compund in association with a solid or liquid pharmaceutically acceptable carrier and, optionally, with pharmaceutically acceptable adjuvants and excipients employing standard and conventional techniques.

The pharmaceutical compositions may be administered orally, parenterally or by rectal suppository. A wide variety of pharmaceutical forms may be employed. Thus, if a solid carrier is used, the preparation may be tableted, placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. If a liquid carrier is employed, the preparation may be in the form of a syrup, emulsion, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. The pharmaceutical compositions are prepared by conventional techniques appropriate to the desired preparation containing appropriate amounts of the active component, that is, the compound of formula I and II according to the invention.

The dosage of the compounds of formula I and II will depend not only on such factors as the weight of the patient and mode of administration, but also on the degree of cholesterol biosynthesis inhibition desired and the potency of the particular compound being utilized. The decision as to the particular dosage to be employed (and the number of times to be administered per day) is within the discretion of the physician, and may be varied by titration of the dosage to the particular circumstances of this invention for the satisfactory inhibition or reduction of cholesterol biosynthesis, each oral dosage unit will contain the active ingredient in an amount of from about 0.01 mg/kg to about 10 mg/kg body weight, and most preferably from about 0.05 mg/kg to about 2 mg/kg body weight. The active ingredient will preferably be administered in equal doses from one to four times a day. However, usually a small dosage is administered, and the dosage is gradually increased until the optimal dosage for the host under treatment is determined.

The following examples and preparations describe the manner and process of making and using the preferred embodiments of the invention and are illustrative rather than limiting. It should be understood that there may be other embodiments which fall within the spirit and scope of the invention as defined by the claims appended hereto.

### Example 1

[4R-[4a,6b(E)]]-6-[4,4-Bis(4-fluorophenyl)-3-(1-methyl-1H-tetrazol-6-yl)-1,3-butadienyl]-tetrahydro-4-hydroxy-2H-pyran-2-one 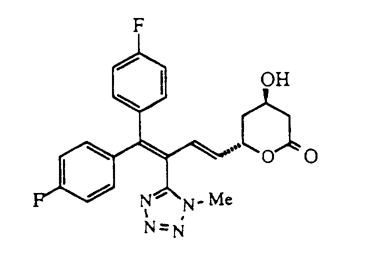 A.[[4R-[4a,6a(E)]]-6-[4,4-Bis(4-fluorophenyl)-3-(1-methyl-1 H-tetrazol-5-yl)-1,3-butadienyl]-2,2-dimethyl-1,3-dioxane-4-acetic acid, 1,1-dimethylethyl ester

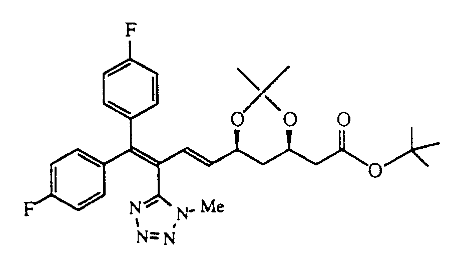

1. 5-[1-Bromomethyl)-2,2-bis(4-flurorphenyl)-ethenyl]-1-methyl-1H-tetrazole

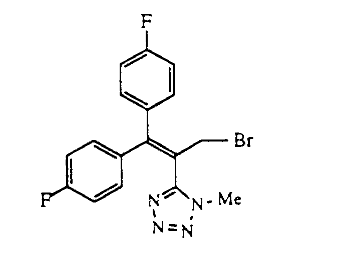

a. 5-[2,2-Bis(4-fluorophenyl)-1-methylethenyl]-1-methyl-1H-tetrazole

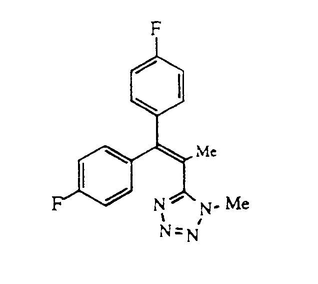

A THF (45 mL) solution of 93.6% pure 5-ethyl-1-methyl-1 H-tetrazole (26.7 g, 222.9 mmol) was added to a cooled (0 to 10_{°}C) THF (270 mL) solution of 2M lithium diisopropyl amide (117 mL, 234 mmol) maintaining the temperature in the range 0 to 10°C. The resulting solution was stirred at 0 to 10°C for 30 minutes and was added to a cooled (0 to 10°C) THF (315 mL) solution of bis(4-fluorophenyl)methanone (53.8 g, 246.4 mmol) maintaining the temperature in the range 0 to 10°C. The reaction was stud at 0 to 10°C until it was judged complete by HPLC or GC. Upon completion of the condensation, the reaction mixture was quenched by the addition of saturated ammonium chloride solution (100 mL) maintaining the temperature in the range 0 to 10°C. Xylenes (475 mL) were added, the mixture was warmed to ambient temperature, and the phases were separated. The organic layer was washed succesively with 1N HCI (2 X 300 mL) and water (2 X 200 mL). The organic layer was concentrated to a volume of about 750 mL and the distillation was continued keeping the pot volume in the range 720 to 750 mL until the pot temperature was in the range 138 to 144°C. The resulting xylenes solution was cooled to 30°C and Amberlyst 15 resin (7.3 g) was added. The reaction flask gas equipped with a Dean-Stark trap and reflux condenser. The xylenes solution was heated at reflux, collecting water in the Dean-Stark trap, until the dehydration reaction was judged complete by GC. The resulting mixture was diluted with THF (188 mL), the resin was collected on a filter and the filtrate was concentrated to a volume of 220 to 240 mL. The distillation was continued maintaining the pot volume of 220 to 240 mL until the pot temperature was in the range of 138 to 144°C. The solution was cooled to ambient temperature, held at ambient temperature for one hour and then cooled to 0 to 10 °C. After stirring the product slurry at 0 to 10°C for about one hour, the product was filtered and the wet cake washed with cold xylenes (95 mL). The product was dried in vacuo at 55 to 60 °C to constant weight to afford crystalline title compound (43 g, 62 M%).

### b. 5-[1-Bromomethyl)-2,2-bis(4-flurorphenyl)-ethenyl]-1-methyl-1H-tetrazole

A solution of 5-[2,2-bis(4-fluorophenyl)-1-methylethenyl]-1-methyl-1 H-tetrazole (48.86 g 156.4 mmol, the title a compound) and NBS (29.29 g, 164.6 mmol) in methylene chloride (275 mL) in a 500 mL round-bottomed flask was placed in a water bath. The reaction mixture was stirred with a magnetic stirrer and irradiated with a 275 watt sunlamp from -11 inches away. The temperature of the bath and reaction mixture were monitored and the progress of the reaction was followed by TLC analysis. At the end of the reaction distilled water (125 mL) was added and the reaction mixture was stirred vigorously for five minutes. Additional water (175 mL) was added, mixed, and the layers were separated. The aqueous phase was extracted with methylene chloride (3 x 40 mL). The organic phases were combined, washed (2 x 300 mL saturated aqueous sodium chloride), dried (anhydrous magnesium sulfate), filtered, and concentrated to give a beige solid (-60 g). This solid was dissolved in boiling ethyl acetate (145 mL) and heptane (160 mL) was added while maintaining the boiling temperature and then the mixture was set aside. After standing at room temperature for -14 hours, it was placed in the cold room (5°C) for five hours. The crystals were filtered and washed with cold heptane (3 x 50 mL) to give rectangular cubic crystals (43.45 g, 71% yield). The mother liquor was combined with the heptane washings and concentrated. The beige solid (-18g) was dissolved in boiling ethylacetate (45 mL), diluted with heptane (50 mL), and then the solution was set aside at room temperature for 15.5 hours. The crystals were filtered and washed with cold heptane (2 x 15 mL) to give a second crop (14.06 g. 23% yield).

### Alternate procedure for preparation of the title 1 compound: 5-[1-Bromomethyl)-2,2-bis(4- flurorphenyl)- ethenyl]-1- methyl- H- tetrazole

A methylene chloride (8.0 L) solution of 5-[2,2-bis(4-fluorophenyl)-1-methylethenyl]-1-methyl-1 H-tetrazole (400 g, 1.28 mol; the title a compound), 1,3-dibromo-5,5-dimethylhydantoin (230 g, 0.80 mol) and di(4-tert-butylcyclohexyl)peroxydicarbonate, PERKADOXO, (6.4 g, 0.016 mol) was pumped through a heat exchanger to increase the temperature of the solution to 30 to 35 _{°} C. The warmed reaction solution was then passed through a photochemical reactor, equipped with a 1200 Watt UV lamp. As the reaction solution exited the photochemical reactor, it was about 98% complete (judged by in-process HPLC analysis of the reaction stream) and was added to an agitated solution of sodium bisulfite (200 g, 1.94 mol) dissolved in water (2.0 L). After all of the reaction solution was passed through the heat exchanger and photochemical reactor into the sodium bisulfite solution, agitation was discontinued and the methylene chloride layer was separated from the sodium bisulfite solution. A portion of the methylene chloride layer (0.45 L) was washed with a saturated solution of sodium bicarbonate (0.5 L) followed by three water washes (0.45 L each). The wet methylene chloride solution was concentrated under reduced pressure and replaced with ethyl acetate. The resulting ethyl acetate solution was concentrated under reduced pressure to 100 to 125 mL and warmed to 70 °C. Heptane (120 to 140 mL) was added slowly, maintaining the temperature around 70 to 72 °C. After the addition was complete, the temperature of the stirred product slurry was held at 70 to 72 °C for 30 minutes, cooled to 22 to 30 _{°} C and stirred for 60 to 90 minutes and then cooled to 0 to 5 _{°} C and held at this temperature for 60 to 75 minutes. The product slurry was filtered, washed with cold heptane (3 X 25 mL) and dried in vacuo to afford crystalline product (20.8 g, 78.5 M% corrected for input volume). Preferably, vacuum drying may be omitted and the heptane wet product can be used in the next step.

2. [3,3-Bis(4-fluorophenyl)-2-(1-methyl-iH-tetrazol-5-yl)-2-propenyl]-phosphonic acid, dimethyl ester

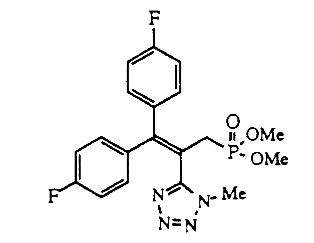

A solution of 5-[1-bromomethyl)-2,2-bis(4-fluorophenyl)ethenyl]-1-methyl-1H-tetrazole (43.26 g, 103.4 mmol, the title 1 compound) in toluene (275 mL) was brought to a boil. To this refluxing solution was added a solution of trimethyl phosphite (13.4 mL, 14.11 g, 113.7 mmol) in toluene (35 mL) in a dropwise fashion over 30 minutes with stirring. The reaction appeared to be complete within two hours by TLC analysis. The reaction mixture was cooled to 55°C and partially concentrated to a total of -100 mL of solution. The solution was brought to a boil to get all material into solution and 1 volume equivalent of heptane (95 mL) was added while maintaining the boiling temperature. It was set aside at room temperature for 65 hours and the crystals were filtered, washed with cold heptane (2 x 100 mL) and dried under vacuum at 62 °C for 17 hours to obtain a shiny white solid (41.49 g, 96% yield).

### Alternate preparation of the title 2 compound: [3,3-Bis(4-fluoro-phenyl)-2-(1-methyl-1H-tetrazol-5-yl)-2-propenyl]-phosphonic acid, dimethyl ester

Heptane wet 5-[1-(bromomethyl)-2,2-bis(4-fluorophenyl)-1-ethenyl]-1-methyl-1 H-tetrazole (88.5 g, 0.226 mol; the title 1 compound) was suspended in toluene (400 mL) and the mixture was concentrated at atmospheric pressure until about 90 mL of distillate had been collected. Under reflux, trimethyl phosphite (29.3 mL, 0.248 mol) was added over 30 minutes to the toluene solution. The reaction mixture was maintained at reflux until it was deemed complete by HPLC assay. The reaction mixture was concentrated at atmospheric pressure until about 90 mL of distillate had been collected and was then cooled to 70 to 80 °C. Heptane (400 mL) was added to the resulting product solution maintaining the temperature at 70 to 80 °C. The temperature of the resulting crystal slurry was cooled to 0 to 5 °C, the product was filtered, the wet cake was washed with cold heptane (100 mL) and dried in vacuo at 45 to 50 °C to afford white crystalline 3,3- bis(4-fluorophenyl)-2-(1-methyl-1 H-tetrazole-5-yl)-2 propenyl]-phosphonic acid, dimethyl ester (89.8 g, 94.5 M%).

### Alternate preparation of the title 2 compound: [3,3-Bis(4-fluorophenyl)-2-(1-methyl-1H-tetrazol-5-yl)-2-propenyl]-phosphonic acid, dimethyl ester

Dimethyl phosphite (7.74 g, 70.3 mmol) was dissolved in tetrahydrofuran (50 mL) and cooled to between -40 °C and -50 °C. Lithium bis(trimethylsilyl)amide in THF (1.0 M solution, 70.3 mL, 70.3 mmol) was added at a rate which maintained the temperature of the reaction mixture between -40 * C and -50 °C and stirred for 15 minutes. The temperature of the reaction mixture was lowered to -75 °C to -78 °C and a THF (100 mL) solution of 5-[1-(bromomethyl)-2,2-bis(4-fluorophenyl)-1-methyl-ethenyl]-1-methyl-1H-tetrazole (25.0 g, 63.9 mmol; the title 1 compound) was added maintaining the temperature between -78 °C and -70 °C. After the addition was complete, the reaction was stirred at -78 to -70 °C until it was deemed complete by HPLC. The reaction was allowed to warm to -10°C and then quenched by the addition of water (75 mL). The THF was removed by distillation, ethyl acetate (250 mL) was added and the rich organic layer was separated from the lower aqueous phase. The rich organic layer was washed with water (50 mL) and separated. The rich ethyl acetate soution was concentrated at atmospheric pressure to the crystallization volume (50 to 60 mL). The ethyl acetate solution was held at 70 °C to 80 °C while heptane (120 to 140 mL) was added to initiate crystallization. The product slurry was allowed to slowly cool to ambient temperature, held for one hour and then cooled to 0 to 5°C. Isolation by vacuum filtration, washing with heptane (50 mL) and drying in vacuo at 45 to 50 °C afforded white crystalline 3,3-bis(4-fluorophenyl)-2-(1-methyl-1 H-tetrazole-5-yl)-2-propenyl]-phosphonic acid, dimethyl ester (23.5g, 88.0 M%).

3. (4R-cis)-6-Formyl-2,2-dimethyl-1,3-dioxolan-4-acetic acid, 1,1-dimethylethyl ester

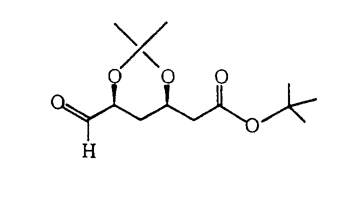

To a magnetically stirred homogeneous solution of oxalyl chloride (21.0 mL, 0.24 mol) in methylene chloride (500 mL) under argon was added a solution of dimethyl sulfoxide (34.0 mL, 0.48 mol) in methylene chloride (150 mL) at -73 °C to -63 °C over a 60 minutes period. After the addition, the reaction mixture was stirred another 10 minutes at -60 °C. A homogeneous solution of (4R-cis)-6-hydroxymethyl-2,2-dimethyl-1,3-dioxolan-4-acetic acid, 1,1-dimethylethyl ester (50.0 g, 0.19 mol) in methylene chloride (150 mL) was added dropwise to the reaction mixture at -65 °C to -55 °Cover 60 minutes. The resulting milky heterogeneous mixture was stirred at -60 °C to -50 °C for additional 15 minutes. Then triethylamine (135 mL) was added dropwise at -60 °C in 30 minutes. After stirring at -60 °C to -63 °C for 60 minutes the milky thick heterogeneous mixture was quenched by pouring into a mixture of ice-water (960 mL) and hexane (720 mL) with vigorous stirring. The mixture was stirred for 15 minutes and the layers were separated and the aqueous layer extracted with hexane (700 mL x 1). The organic phases were combined and washed with cold 5% sodium phosphate monobasic [pH - 4.3; 3x700 mL], brine, dried over magnesium sulfate, filtered and concentrated under reduced pressure. It was further dried under high vacuum (- 0.2 mm Hg) at room temperature to afford 44.8 g of crude aldehyde as a light yellow waxy solid in 90% yield. It was directly used in the next reaction without any further purification: m.p. 56-58°C; TLC; Rₜ = 0.42 [Silica gel, i-PrOH:Hexanes. 15:85, PMA visualization].

### Alternate procedure for preparing the title 3 compound: (4R-cis)-6-Formyl-2,2-dimethyl-1,3-dioxolan-4-acetic acid, 1,1-dimethylethyl ester

To a solution of oxalyl chloride (4.4 mL, 50.0 mmol) in dichloromethane (100 mL) at -70 °C under an argon atmosphere was added a solution of dimethylsulfoxide (7.1 mL, 100.0 mmol) in dichloromethane (7 mL) dropwise over a period of approximately 30 minutes. The internal temperature never exceeded -55°C during this addition. The resultant solution was stirred between -60 and -70 °C for an additional five minutes. A dichloromethane (30 mL) solution of alcohol, (4R-cis)-6-hydroxymethyl-2,2-dimethyl-1,3-dioxolan-4-acetic acid, 1,1-dimethylethyl ester (10.0 g, 38.5 mmol) was slowly added dropwise over a period of approximately 20 minutes (internal temperature kept below -50 °C during the addition). After this addition, the solution was stirred between -55 and -65°C for an additional 15 minutes. Triethylamine (26.8 mL, 192.3 mmol) was then slowly added resulting in the formation of a brilliant, white, heterogeneous mixture. After stirring between -60 and -70 °C for 45 minutes, the reaction was gradually warmed to -40 °C (approximately 30 minutes warming time). It was stirred at -40 °C for another 15 minutes. The reaction was slowly poured into a mixture of ice water (200 mL) and hexane (150 mL) with vigorous stirring. After stirring 15 minutes the aqueous layer was separated and extracted with hexane (60 mL). The organic phases were combined and washed with 5% aqueous sodium phosphate monobasic solution (pH = 4.3; 168 mL x 3), brine (120 mL x 1), dried over anhydrous magnesium sulfate, filtered, and concentrated to dryness at reduced pressure (approx. 1 mm Hg) to produce 9.2 g of a white, waxy solid (93% yield).

### Alternate procedure for preparing the title 3 compound: (4R-cis)-6-Formyl-2,2-dimethyl-1,3-dioxolan-4-acetic acid, 1,1-dimethylethyl ester

Dimethyl sulfoxide, DMSO (34 mL, 480.8 mmol) was dissolved in methylene chloride (500 mL) and cooled to -70 °C to -76 °C under argon. Oxalyl chloride (21.8 mL, 250 mmol) was added dropwise to the clear solution maintaining the temperature at -70 ° C to -76 °C. After stirring the reaction mixture for 30 to 45 minutes, a methylene chloride (150 mL) solution of (4R-cis)-6-hydroxymethyl-2,2-dimethyl-1,3-dioxolan-4- acetic acid, 1,1-dimethylethyl ester (50.0 g, 192.3 mmol), was added dropwise to the reaction mixture at -70 °C to -76 °C. After stirring the reaction mixture for one hour triethylamine (134 mL, 961.5 mmol) was added maintaining the reaction temperature at -70 °C to -76 °C. The reaction was stirred until it was considered complete as judged by in-process GC analysis. The reaction was quenched by the addition of an aqueous solution of 20% monobasic sodium phosphate (50 mL, pH 4.3) at -70 °C to -20 °C followed by the addition of water (500 mL) and heptane (1000 mL) at -20 °C to 25 °C. The biphasic mixture was stirred for 15 minutes, the organic phase was separated and washed with an aqueous solution of 10% monobasic sodium phosphate (3 X 1000 mL, pH 4.3). The organic phase was washed with water (1000 mL) and the organic phase was concentrated under reduced pressure at 30 °C to 45 °C to about 500 mL. Distillation was continued maintaining the pot volume at 450 to 500 mL until the water content of the product solution was less than 0.04%. The quantity of product in the solution was determined and the solution volume was adjusted so that the concentration of product in the solution was about 100 mg/mL. Dilution with heptane afforded a solution which contained 46.3 g of (4R-cis)-6-formyl-2,2-dimethyl-1,3-dioxolan-4-acetic acid, 1,1-dimethylethyl ester (93 M%, solution concentration = 105 mg/mL).

### 4. [[4R-[4a,6a(E)]]-6-[4,4-Bis(4-fluorophenyl)-3-(1-methyl-1H-tetrazol-5-yl)-1,3-butadienyl]-2,2-dimethyl-1,3-dioxane-4-acetic acid, 1,1-dimethylethyl ester

To a 2-liter-3-neck Morton flask, pre-rinsed with dry tetrahydrofuran (200 mL and 100 mL to KF=0.019V%), maintained under a nitrogen atmosphere and equipped with a mechanical stirrer, an internal thermocouple, a claisen adapter and an addition funnel, was charged 40.21 g of the title 2 compound (95.67 mmol) and tetrahydrofuran (345 mL). To the addition funnel was charged a heptane solution (250 mL) containing the title 3 compound (23.31 g; charged based on potency of 93.25 mg/mL, 90.25 mmol) and tetrahydrofuran (233 mL). The flask was cooled to -53°C and KOtBu (100.5 mL; 1.0M in tetrahydrofuran, 100.5 mmol) was added via syringe over ten minutes, while maintaining an internal temperature of ≤-46 °C. The reaction mixture was allowed to cool to -51 _{°} C over ten minutes and then slowly over 20 minutes. The title 3 compound in tetrahydrofuran/heptane was added (with a 20 mL tetrahydrofuran rinse of the addition funnel), T_{max.}=-43°C. The reaction was complete (no title 3 compound visible by PMA char) by TLC (1:1 hexane:ethyl acetate) at T_{Rxn} =five minutes. The reaction was quenched at TRxn=15 minutes by the rapid addition of 25% ammonium chloride (aq) (116 mL). After quenching, the cooling bath was removed, the nitrogen atmosphere was no longer maintained and water (466 mL) and heptane (41 mL) were added. The reaction was allowed to warm to 13°C and the phases were separated. The organic phase was washed with water (2 x 291 mL). The organic phase was reduced to an oil by rotary evaporation, methanol (300 mL) was added and the solution was concentrated to a yellow foam by rotary evaporation. The foam was dissolved in methanol (450 mL) and the resulting solution was stirred magnetically under nitrogen for 12 hours. The resulting precipitates were removed by vacuum filtration through a pad of hyflo (4.25 x 1 cm) with a methanol (80 mL) rinse. The resulting clear yellow solution contained the title compound (47.83 g, 96M%) by HPLC.

### Alternate Preparation of the title A compound: [[4R-[4a,6a(E)]]-6-[4,4-bis(4-fluorophenyl)-3-(1-methyl-1H-tetrazol-5-yl)-1,3-butadienyl]-2,2-dimethyl-1,3-dioxane-4-acetic acid, 1,1-dimethylethyl ester

A solution of K-t-Amylate in toluene (72.67 mL, 1.806 M, 131.25 mmol) was added dropwise over a period of 30 minutes to a mechanically stirred solution of phosphonate, [3,3-bis(4-fluorophenyl)-2-(1-methyl-1 H-tetrazol-5-yl)-2-propenyl]-phosphonic acid, dimethyl ester (57.75 g, 137.50 mmol, the title 2 compound) in tetrahydrofuran (450 mL) under argon. During the addition the internal temperature was maintained at - - 50 _{°} C. The resulting orange colored solution was stirred at -50 _{°} C for an additional one hour. A solution of (4R-cis)-6-Formyl-2,2-dimethyl-1,3-dioxolan-4-acetic acid, 1,1-dimethylethyl ester (32.25 g, 125 mmol; the title 3 compound) in tetrahydrofuran (100 mL) was added dropwise over a period of 30 minutes to the reaction mixture at ~-50°C. After the addition it was stirred for 30 minutes at -50 °C. Saturated aqueous ammonium chloride solution (100 mL) was added slowly to the reaction mixture. The layers were separated and the aqueous layer was extracted with ethyl acetate (300 mL). The combined organic layers were washed with half saturated brine (250 mL), brine (100 mL), dried over anhydrous sodium sulfate, filtered and concentrated in vacuo to furnish 73.8 g of acetonide, the title compound as an amorphous solid. This product was directly used in the next step without any further purification. TLC: R_{f}= 0.33 (E-olefin) [Silica gel, i-PrOH:Hexanes, 1:9, 2 elutions, visualization by UV and PMA].

### B (3R, 5S, 6E)-9,9-Bis(4-flurorphenyl)-3,5-dihydroxy-8-(1-methyl-1H-tetrazol-5-yl)-6,8-nonadienoic acid 1,1-dimethylethyl ester

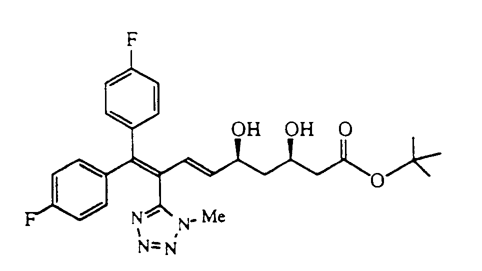

Aqueous 1 N hydrochloric acid (62.5 mL, 62.5 mmol) was slowly added over a period of 10 minutes to a cooled (0°C) and mechanically stirred solution of the acetonide, [[4R-[4a,6a(E)]]-6-[4,4-bis(4-fluorophenyl)-3-(1-methyl-1 H-tetrazol-5-yl)-1,3-butadienyl]-2,2-dimethyl-1,3-dioxane-4-acetic acid, 1,1-dimethylethyl ester (73 g, the title A compound) in methanol (350 mL). After stirring for three hours at room temperature the reaction mixture was cooled to 0°C and neutralized with 1 N sodium hydroxide (65 mL) to - pH 6.8. About 150 mL of methanol was removed from the reaction mixture under reduced pressure on a rotary evaporator at room temperature. The resulting residue contained the title compound.

### Alternate Preparation of the title B compound: (3R,5S,6E)-9,9-Bis(4-flurorphenyl)-3,5-dihydroxy-8-(1-methyl-1H-tetrazol-5-yl)-6,8-nonadienoic acid 1,1-dimethyl-ethyl ester

A cooled (0 to 10°C) methanol solution (650 mL) of [[4R-[4a,6a(E)]]-6-[4,4-bis(4-fluorophenyl)-3-(1-methyl-1 H-tetrazol-5-yl)-1,3-butadienyl]-2,2-dimethyl-1,3-dioxane-4-acetic acid, 1,1-dimethylethyl ester (46.9 g, 84.9 mmol; the title A compound) was treated with 2.4 M HCI (67.3 mL, 161.5 mmol) at 0 to 10°C. The reaction mixture vacuum distilled removing acetone at 5 to 15°C until it was judged complete by HPLC. Methanol was added to maintain a constant volume during the reaction. The acidic methanol/water reaction mixture was neutralized with 1.0 N NaOH (150 mL) to ≈pH=4 then with 0.1 N NaOH (200 mL) to pH=7.0 to 8.0. MIBK (500 mL) was added and the solution was concentrated under vacuum to 400 mL. Additional MIBK (200 mL) was added and the solution was concentrated under vacuum to 500 to 650 mL and the total volume of the product rich solution was adjusted to 700 mL. The layers of the resulting biphasic solution were separated and the MIBK layer was washed with water (200 ML). The rich MIBK phase was concentrated under vacuum to a volume of (-200 mL), and the vacuum distillation was continued maintaining the volume at about ≥200 mL by the addition of fresh MIBK until the solution had a KF of <1 V%. Following HPLC analysis of the resulting solution, the volume was adjusted with MIBK to a total volume of 360 mL with a KF of ≈1.0 V% and then was polish filtered. The solution was heated to 45 to 60 _{°} C and n-heptane (1300 mL) was added while maintaining a temperature of 45 to 60 °C. Crystallization was initiated by seeding with (3R,5S,6E)-9,9-bis(4-fluorophenyl)-3,5-dihydroxy-8-(1-methyl-1 H-tetrazol-5-yl)-6,8-non- adienoic acid, 1,1 dimethylethyl ester, and the slurry was allowed to slowly cool to room temperature over 2 to 3 hours. The resulting crystal slurry was filtered at 0 to 5°C. The wet cake was washed with 9:1 n-heptane/MIBK (200 mL) and then dried on the filter for 30 to 90 minutes. The air dried crude product was obtained as a light yellow crystalline hydrate (38.4 g, 80.4 M%). The air dried crude product was dissolved in 2-propanol (250 mL) and the resulting solution was polish filtered. The filter was rinsed with 2-propanol (30 mL) and the water content of the combined filtrates was adjusted to about 0.8 v% water. Heptane (3100 mL) was added to the warm solution (35 to 50 °C) 2-propanol solution of crude product. After heating the crystallization mixture to about 50 °C, it was cooled to 32 to 36 °C, seeded and agitated for 1 hour. The resulting slurry was cooled to 0 to 5 _{°} C over 2 to 3 hours and agitated at this temperature for 1 hour. The product slurry was filtered, the wet cake was washed with 15:1 n-heptane/2-propanol (2 X 150 mL) and dried under vacuum at 35 to 40 °C. The title compound was obtained as light yellow crystalline hydrate (35.1 g, 73.5 M%).

### C. (3R, 5S, 6E)-9,9-bis(4-fluorophenyl)-3,5-dihydroxy-8-(1-methyl-iH-tetrazol-5-yl)-6,8-nonadienoic acid

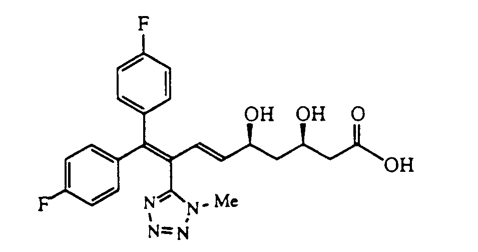

The title B compound was diluted with methanol (430 mL), cooled to 0°C and treated with 1 N sodium hydroxide (125 mL, 125 mmol) over a period of 10 minutes. The reaction mixture was stirred vigorously at room temperature overnight (-12 hours) and then concentrated to an oily residue on a rotary evaporator under reduced pressure at 40 °C. It was diluted with water (500 mL), cooled (0°C), carefully acidified with vigorous stirring to pH - 6.25 with 1 N hydrochloric acid (100 mL) and extracted with ethyl acetate (3 x 400 mL). The combined organic layers were washed with water (until neutral), brine (100 mL), dried (sodium sulfate), filtered and concentrated on a rotary evaporator under vacuo to afford the crude acid, the title compound in quantitative yield (56.36 g), which was directly used in the next step: TLC: Rf = 0.52 [Silica gel, methanol:methylene chloride 3:7, PMA and UV visualization].

### D. (3R,5S,6E)-9,9-Bis(4-fluorophenyl)-3,5-dihydroxy-8-(1-methyl-1H-tetrazol-5-yl)-6,8-nonadienoic acid, cinchonidine (1:1) salt

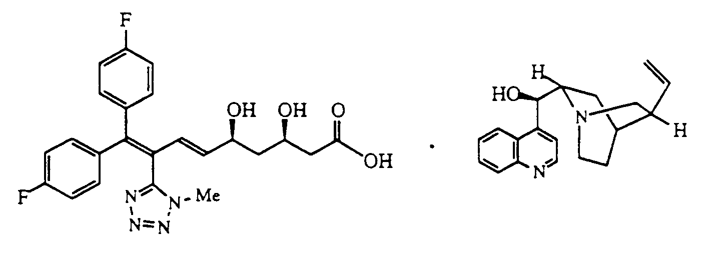

Cinchonidine (11.69 g, 39.71 mmol, 0.995 eq) was added to the washed rich methyl isobutyl ketone solution of the title C compound which was heated to 68 °C to give complete dissolution of all solids. Then heptane (83 mL) was added, initiating crystallization. The thickening suspension was cooled to 60°C and held for ca. one hour. The suspension was gradually cooled to 10°C and held for one hour. The suspended solids were isolated by filtration and were washed with cold 50% heptane in methyl isobutyl ketone (2 x 30 mL) and dried in vacuo at 60 _{°} C to 65 _{°} C to afford the title compound (24.55 g) as a crystalline white solid.

### E. [4R-[4a,6b(E)]]-6-[4,4-Bis(4-fluorophenyl)-3-(1-methyl-1H-tetrazol-5-yl)-1,3-butadienyl]-tetrahydro-4-hydroxy-2H-pyran-2-one

To a clear solution of the title D compound (50 g, 66.83 mmol) and pyridine (16 mL, 3.0 eq, 200.5 mmol) in methylene chloride (900 mL) at -24°C was added dropwise with vigorous stirring trimethylacetyl chloride (9.88 mL, 1.2 eq, 80.2 mmol) over a period of six minutes. The internal temperature was maintained at -24 °C to -21 °C during the addition. The reaction was then stirred between -18°C and -15°C for two and a half hours. It was recooled to -22 °C. To the slightly cloudy solution was added triethylamine (20.49 mL, 147.0 mmol) and the resulting honogeneous solution was poured into a pH 7 buffer solution (Na₂HP0₄., 200 mL). The aqueous layer was separated and extracted with methylene chloride (60 mL x 2). The combined organic layer was washed with 10% NaH₂P0₄ (pH 4.5, 100 mL). A serious emulsion was encountered. The emulsion was broken by the addition of 1N hydrochloric acid (400 mL). The resulting aqueous layer was separated and extracted with methylene chloride (100 mL x 2). The combined organic layer was washed with 10% NaH₂P0₄ (pH 4.5, 100 mL x 3), brine (100 mL x 2), dried over magnesium sulfate, filtered, and concentrated to give a crude lactone (34 g). The crude lactone was dissolved in ethyl acetate (100 mL) and set aside at room temperature for one hour and then at 4°C for 16 hours. The solid was filtered and washed with 50% ethyl acetate in hexane (200 mL), hexane (100 mL), and dried in vacuo - (1 mm Hg) to give the title compound (25 g, 84%).

### Alternate Preparation of the title E compound: [4R-[4a,6b(E)]]-6-[4,4-Bis(4-fluorophenyl)-3-(1-methyl- 1H- tetrazol- 5- yl)-1, 3- butadienyl]- tetrahydro- 4- hydroxy- 2H-pyran- 2- one

The title D compound (8.84 g of title D compound containing 4.83 g of (3R,5S,6E)-9,9-bis(4-fluorophenyl)-3,5-dihydroxy-8-(1-methyl-1 H-tetrazol-5-yl)-6,8-nonadienoic acid, 10.59 mmol) was suspended in ethyl acetate (106 mL) and pyridine (2.57 mL, 31.77 mmol). The resulting suspension was cooled to -20 to -26°C and pivaloyl chloride was added slowly while maintaining the temperature in the aforementioned range. After stirring the reaction mixture at -20 to -26 _{°} C, the suspension dissolves and cinchonidine hydrochloride begins to precipitate. The reaction mixture was warmed to 20 to 25 °C over 2.5 hours and stirred until the reaction was complete as judged by TLC (95:5:2 Ethyl acetate:methanol:acetic acid, visualization UV light) or HPLC. The reaction mixture was filtered, the precipitate was washed with ethyl acetate (2 X 20 mL) and water (20 mL) was added to the combined filtrates. The pH of the biphasic mixture was adjusted to 1.9 by the addition of 1 N HCI (35.1 mL), the phases were separated and water (20 mL) was added to the ethyl acetate layer. The pH of the biphasic mixture was adjusted to 1.9 by the addition of 0.2N HCI (2.7 mL), the phases were separated and water (20 mL) was added to the ethyl acetate layer. The pH of the biphasic mixture was adjusted to 6.8 by the addition of saturated sodium bicarbonate solution (15 mL) stirred for 10 minutes (pH increased to 7.3 to 7.9), the phases were separated and water (20 mL) was added to the ethyl acetate layer. The pH of the biphasic mixture was adjusted to 6.8 by the addition of saturated sodium bicarbonate solution (2.8 mL), stirred for 10 minutes (pH increased to 7.3 to 7.9) and the phases were separated. The ethyl acetate layer was washed with saturated sodium chloride solution (20 mL) and concentrated under vacuum until the water content of the ethyl acetate solution was <0.07%. The concentration was continued until the volume of the solution was 29.6 mL and acetone (10.6 mL) was added. The resulting mixture was polish filtered at 35 to 45°C and wash the filter with 2:1 ethyl acetate/acetone (-16 mL). The combined filtrates were warmed to 43 to 49°C and heptane (45 mL) was added to the combined filtrates until crystallization began. The resulting product slurry was stirred at 43 to 49 °C for 1 hour and then cooled to 5 to 11 _{°} C over 2 hours. The slurry was stirred at 5 to 11 _{°} C for 30 minutes and filtered. The wet cake was washed with cold (-10°C) 1:1 ethyl acetate/heptane (14 mL) and dried under vacuum at 30 to 40 °C to constant weight to afford the title compound as a white crystal (4.05 g, 87.7 M%).

### Example 2

### (3R, 5S, 6E)-9,9-Bis(4-fluorophenyl)-3,5-dihydroxy-8-(1-methyl-1H-tetrazol-5-yl)-6,8-nonadienoic acid

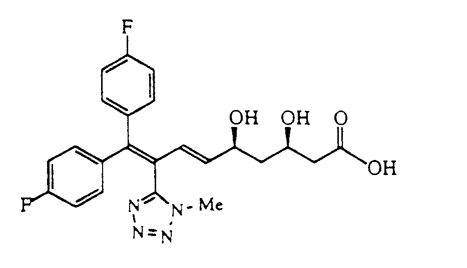

[4R-[4a,6b(E)]]-6-[4,4-Bis(4-fluorophenyl)-3-(1-methyl-1 H-tetrazol-5-yl)-1,3-butadienyl]-tetrahydro-4-hydroxy-2H-pyran-2-one (15 g, 34.2 mmol; the title compound of Example 1), was slurried in water (150 mL) and methanol (20 mL) in a 1 L round-bottomed flask equipped with a mechanical stirrer and dropping funnel. The mixture was stirred for five minutes at 24 °C followed by dropwise addition of a 1.ON solution of sodium hydroxide (34.2 mL). A clear solution resulted upon stirring for three hours at ambient temperature. The solution was assayed by TLC and HPLC and the apparent pH of the mixture was adjusted by dropwise addition of concentrated hydrochloric acid to pH 3.0. Upon acidification below pH 4.2, a white precipitate formed and was dissolved by addition of methyl-t-butyl ether (MTBE, 50 mL). The mixture was stirred for 15 minutes and transferred to a separatory funnel. The layers were separated, and the aqueous phase was extracted with MTBE (250 mL). The combined organic phase was dried over sodium sulfate (anhydrous), filtered, the filter cake washed with MTBE, and the filtrate concentrated to afford the title compound as an oil.

### Example 3

### (3R, 5S, 6E)-9,9-Bis(4-f)uoropheny))-3,5-dihydroxy-8-(1-methy)-1H-tetrazo)-5-y))-6,8-nonadienoic acid

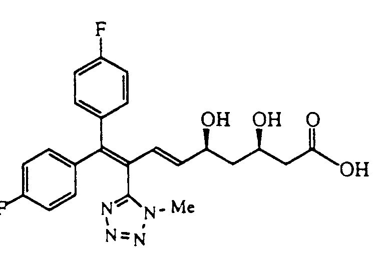

[4R-[4a,6b(E)]]-6-[4,4-Bis(4-fluorophenyl)-3-(1-methyl-1 H-tetrazol-5-yl)-1 ,3-butadienyl]-tetrahydro-4-hydroxy-2H-pyran-2-one; (30 g, 68.4 mmol; the title compound of Example 1), was slurried in water (300 mL) and ethanol (100 mL) in a 1 round-bottomed flask equipped with a mechanical stirrer, thermometer and dropping funnel. The mixture was stirred for five minutes at 24 °C followed by dropwise addition of a 10N solution of sodium hydroxide (13.6 mL) with stirring. A clear solution resulted upon stirring for three hours at ambient temperature. The solution was assayed by TLC and HPLC and the apparent pH of the mixture was adjusted by dropwise addition of concentrated hydrochloric acid to pH 2.25. Upon acidification below pH 4.2, a white precipitate formed and was dissolved by addition of methyl-t-butyl ether (MTBE, 300 mL). The mixture was stirred for 15 minutes and transferred to a separatory funnel with an additional 500 mL MTBE. The layers were separated, and the aqueous phase was extracted with MTBE (500 mL). The combined organic phase was dried over sodium sulfate (anhydrous), filtered, the filter cake washed with MTBE, and the filtrate concentrated to dryness. The white residue (foam) was transferred to a crystallization dish and dried in vacuo at ambient temperature for 12 hours to afford the title compound as a white solid (31.0g, 99M%).

### Example 4

### (3R, 5S, 6E)-9,9-Bis(4-fluorophenyl)-3,5-dihydroxy-8-(1-methyl-iH-tetrazol-5-yl)-6,8-nonadienoic acid

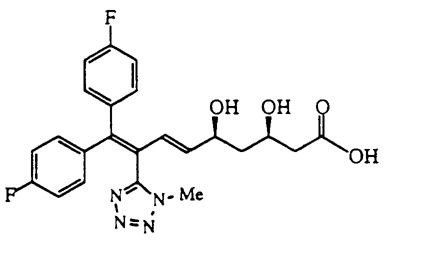

(3R,5S,6E)-9,9-Bis(4-fluorophenyl)-3,5-dihydroxy-8-(1-methyl-1H-tetrazol-5-yl)-6,8-nonadienoic acid, cinchonidine (1:1) salt (8.00 g, 9.14 mmol; the title D compound of Example 1) was suspended in ethyl acetate (32 mL) and water (32 mL). The pH of the biphasic mixture was adjusted to 2.9 ± 0.1 by adding 1 N hydrochloric acid (16 mL). The phases were allowed to separate for at least 30 minutes. Water (32 mL) was added to the upper rich ethyl acetate phase and the pH of the biphasic mixture was adjusted to 2.9 ± 0.1 by adding 1 N hydrochloric acid (0.9 mL). The rich organic was washed with an aqueous sodium chloride solution (15 mL) and ethyl acetate (5 to 7 mL) was used to rinse the transfer vessels and was added to the rich ethyl acetate solution. The rich ethyl acetate solution contained - 9.14 mmol of the title compound.

### Example 5

### (3R, 5S, 6E)-9,9-Bis(4-fluorophenyl)-3,5-dihydroxy-8-(1-methyl-1H-tetrazol-5-yl)-6,8-nonadienoic acid

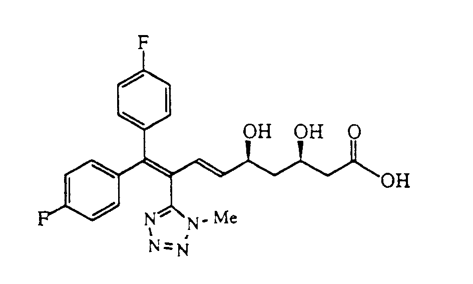

The slightly concentrated reaction mixture containing (3R, 5S, 6E)-9,9-bis(4-fluorophenyl)-3,5-dihydroxy-8-(1-methyl-1H-tetrazol-5-yl)-6,8-nonadienoic acid, 1,1-dimethyl ethylester (20.46 g, 39.92 mmol; the title B compound of Example 1) in methanol was cooled to 21 _{°} C and 10 N sodium hydroxide (3.992 mL, 1.0 eq.) was added, keeping the temperature below 30 °C. The reaction was found to be complete by an in-process HPLC assay run two hours after the addition of the 10 N sodium hydroxide. Water (160 mL) was added and the methanol is removed on a rotavap (167 mL distillate collected), with the internal temperature being kept below 35 _{°} C. The rich aqueous solution containing the sodium salt of the title compound was washed five times with methyl t-butyl ether (45 mL per wash). Methyl isobutyl ketone (135 mL) was added and the pH of the biphasic mixture was lowered to 2.90 by the addition of 1 N hydrochloric acid (41.0 mL, 42.09 g). The spent aqueous was extracted with methyl isobutyl ketone (135 mL). The combined organic phases were washed twice with water (45 mL per wash) and twice with saturated sodium chloride (45 mL per wash) to obtain the title compound (-21.0 g).

### Alternate Preparation of the title compound of Example 5: (3R, 5S,6E)-9,9Bis(4-fluorophenyl)-3,5-dihydroxy-8-(1-methyl-1H-tetrazol-5-yl)-6,8-nonadienoic acid

(3R, 5S, 6E)-9,9-Bis(4-fluorophenyl)-3,5-dihydroxy-8-(1-methyl-lH-tetrazol-5-yl)-6,8-nonadienoic acid 1,1-dimethyl ethylester (10.0 g of hydrate containing 9.61 g anhydrous title B compound of Example 1, 18.75 mmol) was dissolved in THF (20 mL) and 1 N NaOH (19 mL) was added. The resulting solution was stirred at 18 to 25 °C until the reaction was complete as judged by in-process HPLC analysis. THF was removed under vacuum at 20 to 35 _{°} C (pot temperature) and the resulting aqueous solution was diluted with water until the volume of the solution was 60 mL. The crude rich aqueous solution was washed methyl tert-butyl ether (3 X 25 mL). The pH of the aqueous layer was lowered to 6.5 to 8.5 by the addition of 1N HCI (1.5 mL). Ethyl acetate (40 mL) was added and the pH of the biphasic mixture was lowered to 2.8 to 3.0 by the addition of 1 N HCI (18.9 mL). Separate the phases, wash the product rich ethyl acetate layer with 5% aqueous sodium chloride solution (20 mL) and separate the phases to afford the title compound (18.75 mmol).

### Example 6

### (3R,5S,6E)-9,9-Bis(4-fluorophenyl)-3,5-dihydroxy-8-(1-methyl-1H-tetrazol-5-yl)-6,8-nonadienoic acid, (L)-Arginine (1:1) salt

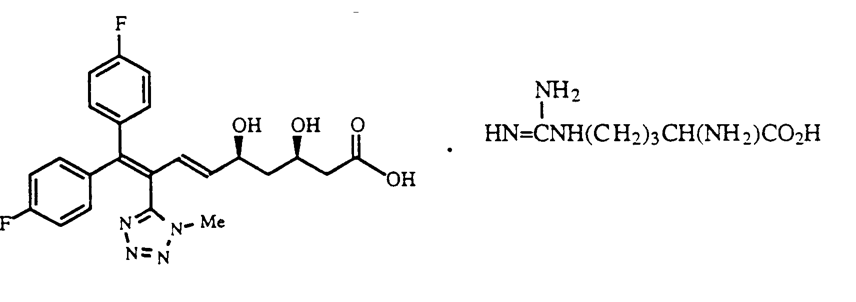

To an ethanol solution (150 mL) of (3R, 5S, 6E)-9,9-bis(4-fluorophenyl)-3,5-dihydroxy-8-(1-methyl-1 H-tetrazol-5-yl)-6,8-nonadienoic acid (15.5 g, 33.9 mmol; the title compound of Example 2, 3, 4 or 5) was added L-arginine (5.95 g, 34 mmol) in small portions over five minutes with vigorous agitation. The mixture was heated to 40°C to 50°C and water (10 mL) was added with stirring over 30 minutes to afford a colorless solution. The solution was treated dropwise with MTBE (200 mL) at 45 °C to 50 °C over one hour, and the crystal slurry was cooled to ambient temperature with stirring. The slurry was cooled in an ice/water bath to 0°C to 5°C over 30 minutes and held for 30 minutes. It was then vacuum filtered and assayed. The filter cake was washed with MTBE (100 mL) and air dried over five minutes. The filter cake was broken up and dried in vacuo at 38°C for 14 hours to afford the title compound as a white crystal (17.6 g, 81.6M%).

### Example 7

### (3R,5S,6E)-9,9-Bis(4-fluorophenyl)-3,5-dihydroxy-8-(1-methyl-1H-tetrazol-5-yl)-6,8-nonadienoic acid, (L)-Arginine (1:1) salt

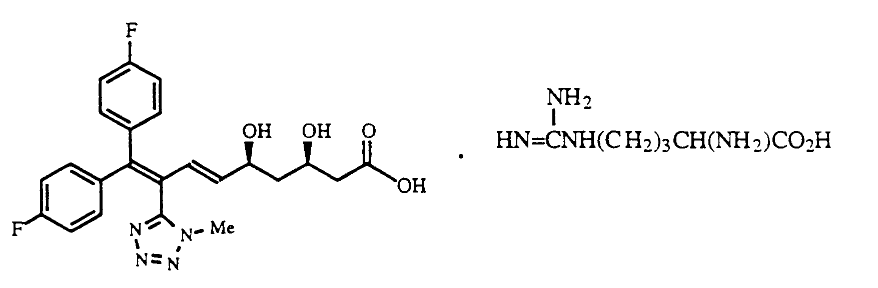

To an ethanol solution (250 mL) of (3R, 5S, 6E)-9,9-bis(4-fluorophenyl)-3,5-dihydroxy-8-(1-methyl-1 H-tetrazol-5-yl)-6,8-nonadienoic acid (9.13 g, 20 mmol; the title compound of Example 2, 3, 4 or 5) was added L-arginine (3.48 g, 20 mmol) in small portions over five minutes with vigorous agitation. The mixture was heated to 40 °C to 50 °C and water (5 mL) was added with stirring over 30 minutes to afford a colorless solution. The solution was treated dropwise with MTBE (1250 mL) at 40 °C to 50 °C over 1.5 hours, and the crystal slurry was cooled to ambient temperature with stirring. The slurry was cooled in an ice/water bath to 0°C to 5°C over 30 minutes and held for one hour. It was then vacuum filtered (Whatman #1)and assayed. The filter cake was washed with MTBE (100 mL) and air dried over five minutes. The filter cake was broken up and dried in vacuo at 35 °C for 12 hours to afford the title compound as a white crystal (11 g, 88M%).

### Example 8

### (3R,5S,6E)-9,9-Bis(4-fluorophenyl)-3,5-dihydroxy-8-(1-methyl-1H-tetrazol-5-yl)-6,8-nonadienoic acid, (L)-Arginine (1:1) salt

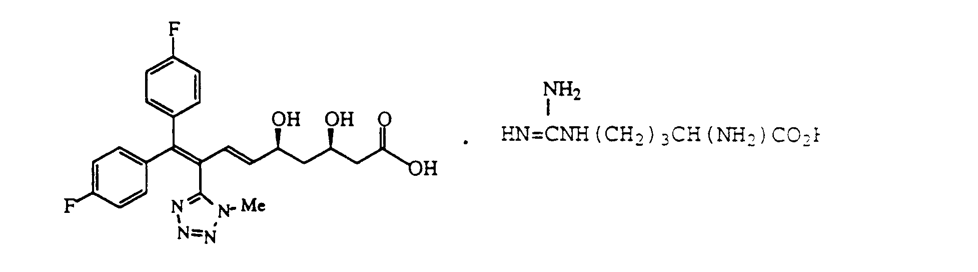

(L)-Arginine (3.27 g, 18.75 mmol) was slurried in ethanol (20 to 22 mL) and an ethyl acetate solution of (3R, 5S,6E)-9,9-bis(4-fluorophenyl)-3,5-dihydroxy-8-(1-methyl-1 H-tetrazol-5-yl)-6,8-nonadienoic acid (18.75 mmol) was added to the (L)-arginine slurry. The addition flask was rinsed with ethyl acetate (20 to 30 mL) and ethanol (10 mL) and both rinses were added to the crystallization mixture. The crystallization mixture was heated to 50 to 60°C and water (7 mL) was added to dissolve solids. Ethyl acetate (100 mL) was added to initiate crystallization while the temperature of the crystallization mixture was lowered to 40 to 50 °C. After crystallization began the product slurry was agitated at 40 to 50 °C for at least one hour and then cooled to 8 to 15°C over about 1.5 hours. The slurry was agitated at 8 to 15°C for at least 30 minutes and the product was collected on a filter. The wet cake was washed with cold (8 to 15°C) 10 v/v% 190 proof ethanol in ethyl acetate (20 to 25 mL) and the product was dried under vacuum at 35 to 45 °C to a constant weight to afford the title compound as white crystalline plates (11.34 g, 91.4 M%).

### Example 9

### (3R,5S,6E)-9,9-Bis(4-fluorophenyl)-3,5-dihydroxy-8-(1-methyl-1H-tetrazol-5-yl)-6,8-nonadienoic acid, 2,6-diamino-hexanoic acid, (L)-lysine (1:1) salt

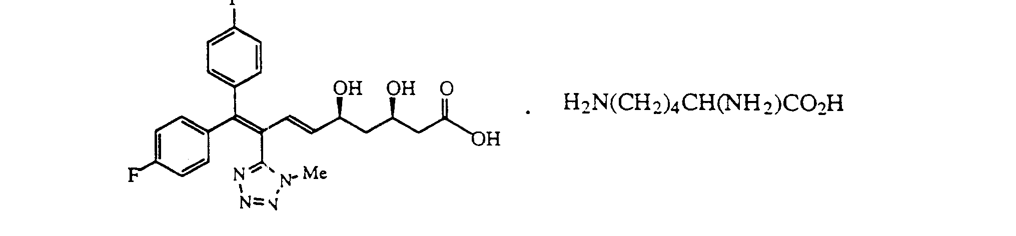

To an ethanol solution (250 mL) of (3R, 5S, 6E)-9,9-bis(4-fluorophenyl)-3,5-dihydroxy-8-(1-methyl-1 H-tetrazol-5-yl)-6,8-nonadienoic acid (9.13 g, 20 mmol; the title compound of Example 2, 3, 4 or 5) was added L-lysine (2.92 g, 20 mmol) in small portions over five minutes with vigorous agitation. The mixture was heated to 40 °C to 50 °C and water (4 mL) was added with stirring over 15 minutes to afford a colorless solution. The solution was treated dropwise with MTBE (750 mL) at 40 °C to 50 °C over one hour, and the crystal slurry was cooled to ambient temperature with stirring. The slurry was cooled in an ice/water bath to 0°C to 5 _{°} C over 30 minutes and held for one hour. It was then vacuum filtered and assayed. The filter cake was washed with MTBE (100 mL) and air dried over five minutes. The filter cake was broken up and dried in vacuo at 35 °C for 12 hours to afford the title compound as a white crystal (7.5 g, 62M%).

## Claims

1. A compound of the formula 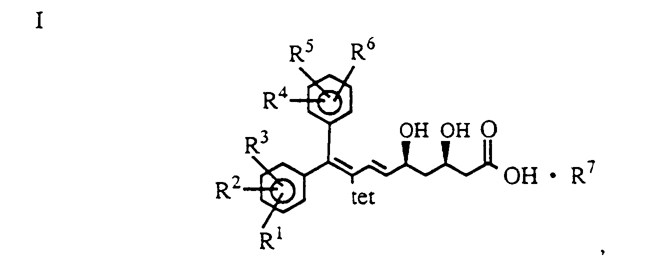
wherein R¹ and R⁴ are independently hydrogen, halogen, C₁ -₄ alkyl, C₁ -₄ alkoxy or trifluoromethyl; R², R³, R⁵ and R⁶ are independently hydrogen, halogen, C₁ -₄ alkyl or C₁ -₄ alkoxy; R⁷ is an amino acid; and tet is 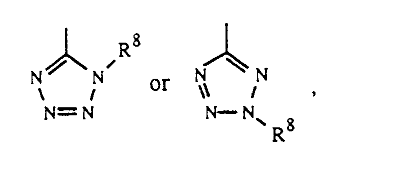
where R⁸ is hydrogen, C₁ -₄ alkyl, C₁ -₄ alkoxyalkyl or (2-methoxy-ethoxy)methyl.

2. The compound of Claim 1 wherein Ri, R₂, R₃, R₄, R₅ and R₆ are independently hydrogen, halogen, C₁ -₄ alkyl or C₁ -₄ alkoxy.

3. The compound of Claim 1 wherein R₁ and R₄ are hydrogen and R₂, R₃, R₅ and R₆ are independently hydrogen, fluoro, chloro, methyl or methoxy.

4. The compound of Claim 1 wherein tet is 1 H-tetrazol-5-yl or 1-substituted-1 H-tetrazol-5-yl.

5. The compound of Claim 2 wherein tet is 1 H-tetrazol-5-yl or 1-substituted-1 H-tetrazol-5-yl.

6. The compound of Claim 1 wherein tet is 1-methyl-1H-tetrazol-5-yl, 1-ethyl-1H-tetrazol-5-yl, 1-methylethyl-1 H-tetrazol-5-yl or 1-(2-methoxyethoxy)-methyl-1 H-tetrazol-5-yl.

7. The compound of Claim 1 wherein tet is 1-methyl-1 H-tetrazol-5-yl.

8. The compound of Claim 5 wherein tet is 1-methyl-1 H-tetrazol-5-yl.

9. The compound of Claim 1 wherein the amino acid is L-arginine.

10. The compound of Claim 8 wherein the amino acid is L-arginine.

11. The compound of Claim 1 wherein the amino acid is lysine.

12. The compound of Claim 8 wherein the amino acid is lysine.

13. A method of preparing compounds of formula 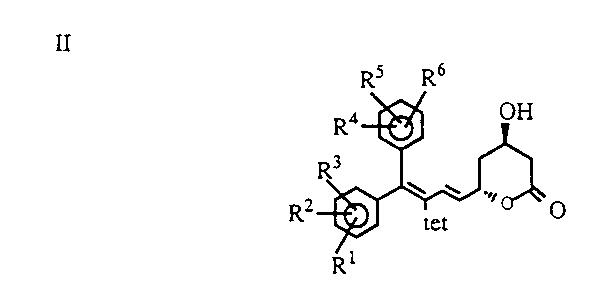
wherein R¹ and R⁴ are independently hydrogen, halogen, C₁ -₄ alkyl, C₁ -₄ alkoxy or trifluoromethyl; R², R³, R⁵ and R⁶ are independently hydrogen, halogen, C₁ -₄ alkyl or C₁ -₄ alkoxy; and tet is 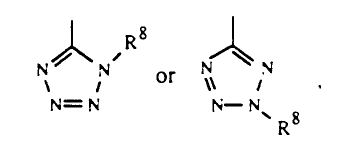
where R⁸ is hydrogen, C₁ -₄ alkyl, C₁ -₄ alkoxyalkyl or (2-methoxy-ethoxy)methyl; comprising the steps of (a) reacting a compound of formula 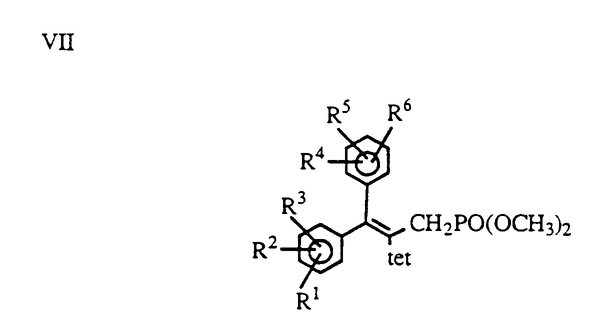
with a compound of formula 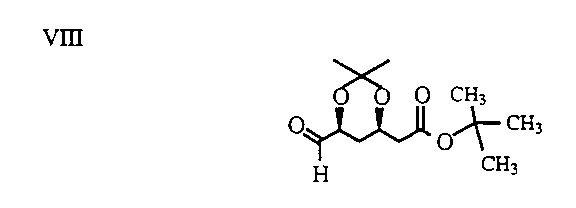
in the presence of a condensation agent to form compounds of formula 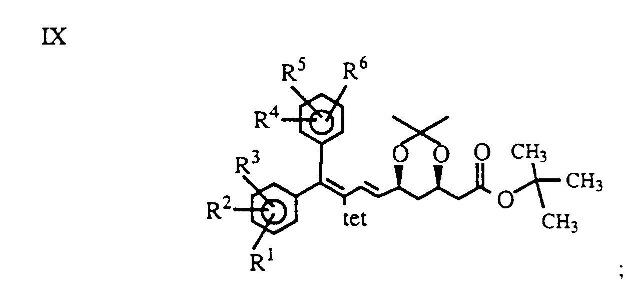
(b) reacting compounds of formula IX with an acid to form a compound of formula 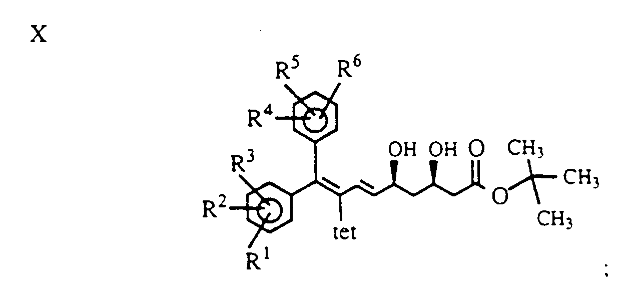
(c) reacting the compound of formula X with a base followed by an acid to form the compounds of formula 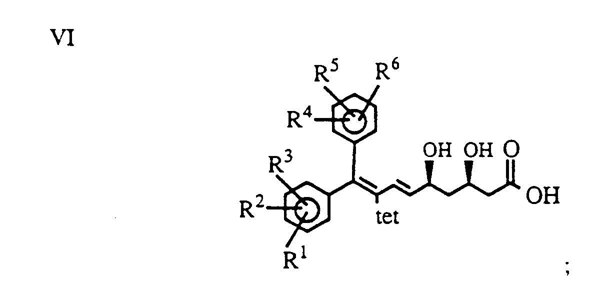
(d) reacting the compound of formula VI with cinchonidine to form a compound of formula 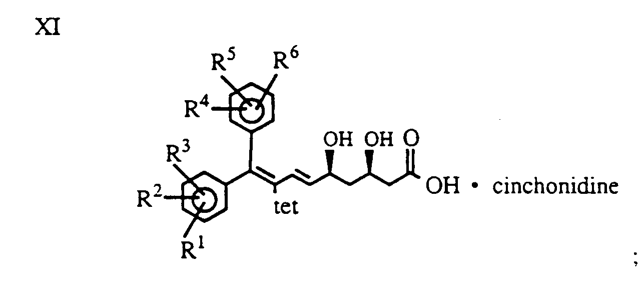 and
(e) reacting the compound of formula XI with a coupling reagent or an activating agent to form the compounds of formula II.

14. The method of Claim 13 wherein the condensation agent is KO-tAm or KO-t-Bu.

15. The method of Claim 13 wherein the coupling reagent is pivaloyl chloride or DCC.

16. A compound of the formula 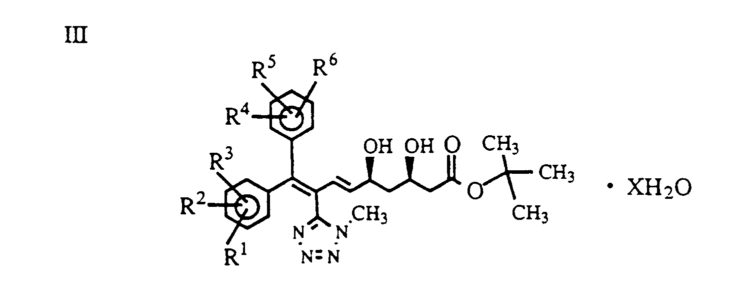
wherein R¹ and R⁴ are independently hydrogen, halogen, C₁ -₄ alkyl, C₁ -₄ alkoxy or trifluoromethyl; R², R³, R⁵ and R⁶ are independently hydrogen, halogen, C₁ -₄ alkyl or C₁ -₄ alkoxy; and X is from about 0.45 to about 0.65.

17. A method of forming compounds of formula 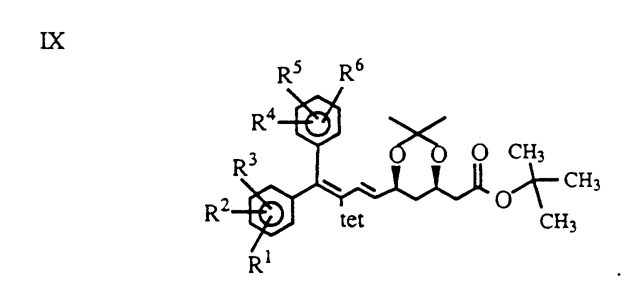
wherein R¹ and R⁴ are independently hydrogen, halogen, C₁ -₄ alkyl, C₁ -₄ alkoxy or trifluoromethyl; R², R³, R⁵ and R⁶ are independently hydrogen, halogen, C₁ -₄ alkyl or C₁ -₄ alkoxy; and tet is 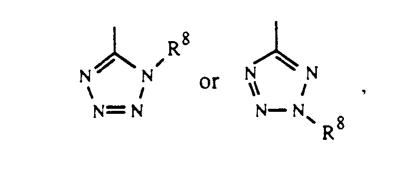
where R⁸ is hydrogen, C₁ -₄ alkyl, C₁ -₄ alkoxyalkyl or (2-methoxy-ethoxy)methyl; comprising the step of reacting a compound of formula 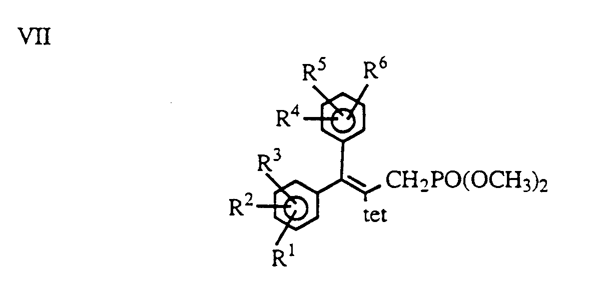
with a compound of formula 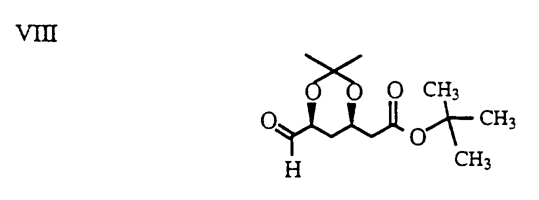
in the presence of a condensation agent in an organic solvent.

18. A method of forming a compound of formula 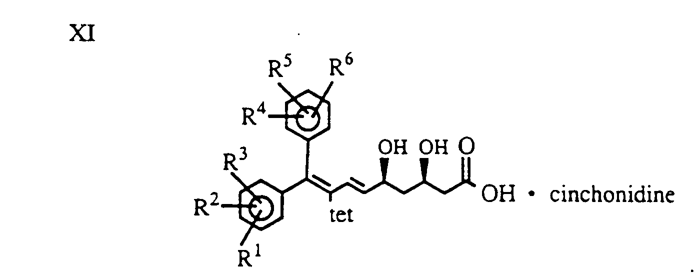
wherein R¹ and R⁴ are independently hydrogen, halogen, C₁ -₄ alkyl, C₁ -₄ alkoxy or trifluoromethyl; R², R³, R⁵ and R⁶ are independently hydrogen, halogen, C₁ -₄ alkyl or C₁ -₄ alkoxy; and tet is 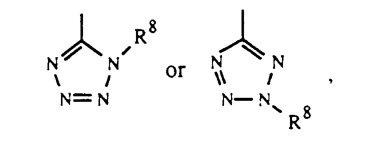
where R⁸ is hydrogen, C₁ -₄ alkyl, C₁ -₄ alkoxyalkyl or (2-methoxy-ethoxy)methyl; comprising the step of reacting compounds of formula 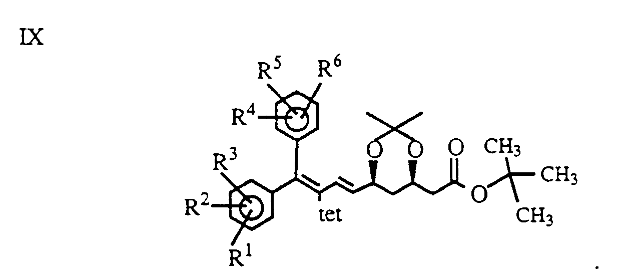
with an acid, followed by a base and then an acid and reacting the product with cinchonidine.

19. A method of forming compounds of formula 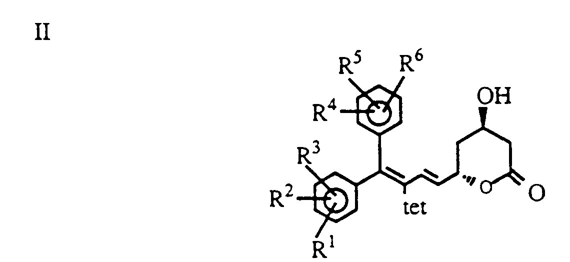
wherein R¹ and R⁴ are independently hydrogen, halogen, C₁ -₄ alkyl, C₁ -₄ alkoxy or trifluoromethyl; R², R³, R⁵ and R⁶ are independently hydrogen, halogen, C₁ -₄ alkyl or C₁ -₄ alkoxy; and tet is 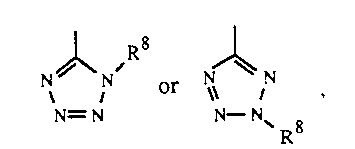
where R⁸ is hydrogen, C₁ -₄ alkyl, C₁ -₄ alkoxyalkyl or (2-methoxy-ethoxy)methyl; comprising the steps of reacting a compound of formula 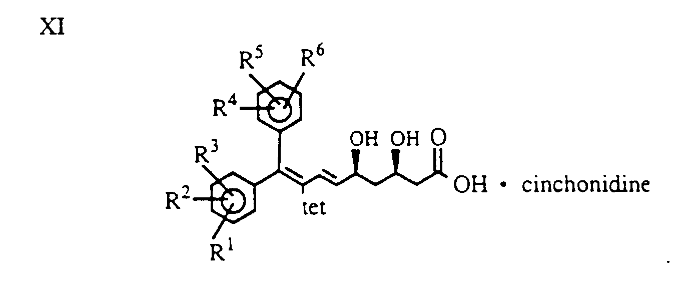
with a coupling reagent or an activating agent.

20. A method for the continuous preparation of compounds of the formula 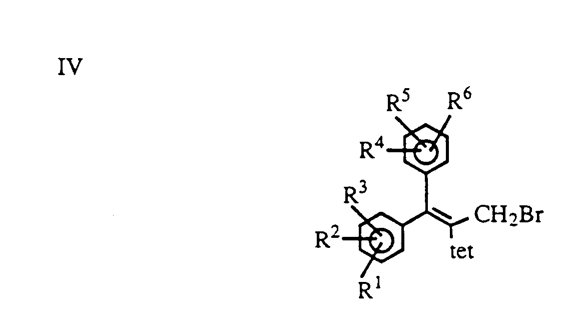
wherein R¹ and R⁴ are independently hydrogen, halogen, C₁ -₄ alkyl, C₁ -₄ alkoxy or trifluoromethyl; R², R³, R⁵ and R⁶ are independently hydrogen, halogen, C₁ -₄ alkyl or C₁ -₄ alkoxy; and tet is 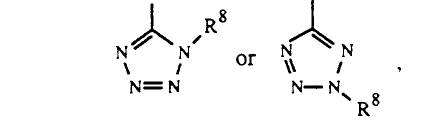
where R⁸ is hydrogen, C₁ -₄ alkyl, C₁ -₄ alkoxyalkyl or (2-methoxy-ethoxy)methyl comprising the step of exposing a continuous stream of a heated solution comprising a compound of formula 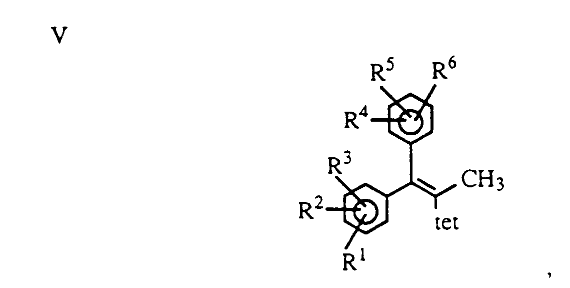
a brominating agent, a free radical initiator and a halogenated hydrocarbon solvent to UV or visible light.

21. The method of Claim 20 wherein the brominating agent is 1,3-dibromo-5,5-dimethylhydantoin.

22. The method of Claim 20 wherein the free radical initiator is di(4-tert-butylcyclohexyl)peroxydicarbonate.

23. The method of Claim 20 wherein the halogenated hydrocarbon solvent is methylene chloride.

24. The method of Claim 20 wherein the heated solution is at a temperature between about 30 °C to 35 °C.
